# EUROPEAN PATENT SPECIFICATION

(11) **EP 3 672 670 B1**
(45) Date of publication and mention of the grant of the patent: **06.10.2021**
(21) Application number: 18750228.1
(22) Date of filing: 13.08.2018
(51) Int. Cl.: A61M 16/00, A41D 13/11, A62B 18/00

(54) **BREATHING MASK AND MASK CONTROL METHOD**
ATEMMASKE UND MASKENSTEUERUNGSVERFAHREN
MASQUE RESPIRATOIRE ET PROCÉDÉ DE COMMANDE DE MASQUE

(30) Priority: 22.08.2017 WO PCT/CN2017/098528; 02.11.2017 EP 17199751
(43) Date of publication of application: 01.07.2020
(73) Proprietor: Koninklijke Philips N.V., 5656 AG Eindhoven (NL)
(72) Inventor: SHI, Jun, 5656 AE Eindhoven (NL); WEI, Huibin, 5656 AE Eindhoven (NL); LUO, Zhongchi, 5656 AE Eindhoven (NL); HILBIG, Rainer, 5656 AE Eindhoven (NL)
(74) Representative: Philips Intellectual Property & Standards
(86) International application number: PCT/EP2018/071846
(87) International publication number: WO 2019/038112

(56) References cited:
- WO-A1-2004/009169
- WO-A1-2016/096775
- SG-A- 2013 097 183
- US-A1- 2007 251 522

## Description

### FIELD OF THE INVENTION

This invention relates to breathing masks.

### BACKGROUND OF THE INVENTION

Air pollution is a worldwide concern. The World Health Organization (WHO) estimates that 4 million people die from air pollution every year. Part of this problem is the outdoor air quality in cities. Nearly 300 smog-hit cities fail to meet national air quality standards.

Official outdoor air quality standards define particle matter concentration as mass concentration per unit volume (e.g. µg/m³). A particular concern is pollution with particles having a diameter less than 2.5 µm (termed "PM2.5") as they are able to penetrate into the gas exchange regions of the lung (alveoli), and very small particles (<100 nm) may pass through the lungs to affect other organs.

Since this problem will not improve significantly on a short time scale, a common way to deal with this problem is to wear a mask which provides cleaner air by filtration and the market for masks in China and elsewhere has seen a great surge in recent years. For example, it is estimated that by 2019, there will be 4.2 billion masks in China.

However, during use, the temperature and relative humidity inside the mask increases and combined with the pressure difference inside the mask relative to the outside, makes breathing uncomfortable. To improve comfort and effectiveness, a fan can be added to the mask which draws in air through a filter. For efficiency and longevity reasons these are normally electrically commutated brushless DC fans.

The benefit to the wearer of using a powered mask is that the lungs are relieved of the slight strain caused by inhalation against the resistance of the filters in a conventional non-powered mask.

Furthermore, in a conventional non-powered mask, inhalation also causes a slight negative pressure within the mask which leads to leakage of the contaminants into the mask, which leakage could prove dangerous if these are toxic substances. A powered mask is able to deliver a steady stream of air to the face and may for example provide a slight positive pressure, which may be determined by the resistance of an exhale valve, to ensure that any leakage is outward rather than inward.

Fan assisted masks thus may improve the wearing comfort by reducing the temperature, humidity and breathing resistance.

Masks may be provided with an inhale fan or an exhale fan or both. An inhale fan assists in drawing air through a filter and enables a positive mask pressure to be obtained. An exhale fan assists in the mask ventilation and ensures the breathed out carbon dioxide is fully expelled.

The position of the inhale and/or exhale fan is typically simulated according to a preset situation of breathing expected during use of the mask, e.g. nasal breathing or oral breathing. However, the breathing mode varies from person to person, and this can result in a decrease of the air exchange efficiency and comfort level.

There is therefore a need for a powered (fan assisted) mask design which provides optimum operational functionality, for different users who may breathe in different ways. SG2013097183 describes respiratory devices for nose and mouth to protect users from dusts and vapors having one or more piezoelectric air pumps.

### SUMMARY OF THE INVENTION

The invention is defined by the claims. Described is a breathing mask comprising:
a mask body for covering the nose and mouth of a user thereby defining a mask chamber;
a filter;
an air pump arrangement for ventilating the mask chamber, wherein the air pump arrangement comprises a first flow area and a second flow area, wherein the flow through the first flow area and the second flow area are independently controllable;
a sensor arrangement for distinguishing between a nasal breathing type and an oral breathing type; and
a controller for controlling the air pump arrangement to provide a flow though the first flow area and/or the second flow area in dependence on the detected breathing type.

This mask design adapts the air pump arrangement (which may be a fan or fans, or a micropump arrangement) to the type of breathing of the wearer of the mask. In particular, the location or flow direction of the air pump flow area is matched to nasal or oral breathing, to improve the comfort of the mask and the effectiveness of the ventilation.

The air pump arrangement is for example for controlling the passage of air into the mask chamber and out of the mask chamber. In this way, the flow is controlled during breathing in and breathing out, and different flow areas may be used for different parts of the breathing cycle, or else the same flow area may be used for both breathing in and out. Thus, the air pump arrangement may be configurable differently as between breathing in and breathing out, if desired.

The sensor arrangement may comprise a plurality of sensor modules, at different locations within the mask chamber. These different locations enable discrimination between nasal and oral breathing. For example one sensor module may be located at a position which, in use of the mask, is for detecting a nasal breath flow and one sensor module is located at a position which, in use of the mask, is for detecting an oral breath flow.

Each sensor module may comprise a temperature sensor and a relative humidity sensor. The breathed out air has a high temperature and higher relative humidity than for the general air content of the mask chamber. For example, the exhale region for the mouth may be a little bit lower than the exhale region for the nose, although they may not be different for all users. Thus, for some people their exhale region for mouth breathing and nose breathing could be similar.

The air pump arrangement may comprise an array of pump channels. This provides a compact pump arrangement. The pump channels are preferably controllable either individually or in groups. For example, the pump channels may be grouped into regions which are then controlled as a single unit.

Each pump channel may for example have controllable pump flow direction. Thus, the single pump channel arrangement functions both as an inhale (inlet) and exhale (exhaust) pump.

The pump channels may for example comprise a first sub-array forming the first flow area and a second sub-array forming the second flow area. One flow area may be in line with the mouth and the other flow area may be in line with the nostrils.

The controller may for example be further adapted to control the air pump arrangement in synchronism with the breathing cycle of the user.

The invention also provides a method of controlling a mask which comprises a mask body for covering the nose and mouth of a user thereby defining a mask chamber and an air pump arrangement for controlling ventilation of the mask chamber, wherein the method comprises:
detecting a nasal breathing type or an oral breathing type; and
controlling the air pump arrangement to provide a flow though a first flow area and/or a second flow area in dependence on the detected breathing type.

This method provides control of the direction or location of a pump flow depending on the detection of nasal breathing or oral breathing.

The method may comprise controlling the passage of air into the mask chamber and out of the mask chamber, so that both inhale and exhale ventilation is provided.

The air pump arrangement may comprise an array of pump channels and controlling the air pump arrangement comprises controlling the activation and flow direction of the pump channels. The air pump arrangement is for example controlled in synchronism with the breathing cycle of the user.

The invention may be implemented at least in part in software.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
- Fig. 1: shows a mask in accordance with the invention;
- Fig. 2: shows one example of an air pump layout with two flow areas;
- Fig. 3: shows one design of air pump with an array of micropump channels;
- Fig. 4: shows the components of the mask of Fig. 1; and
- Fig. 5: shows a mask control method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breathing assistance mask in which an air pump arrangement (e.g. fan or microchannel pump) is used for ventilating the mask chamber. The mask distinguishes between a nasal breathing type and an oral breathing type, and the air pump arrangement is controlled accordingly to provide an appropriate flow location and/or direction. In this way, the operation of the mask is effective regardless of the type of breathing of the user.

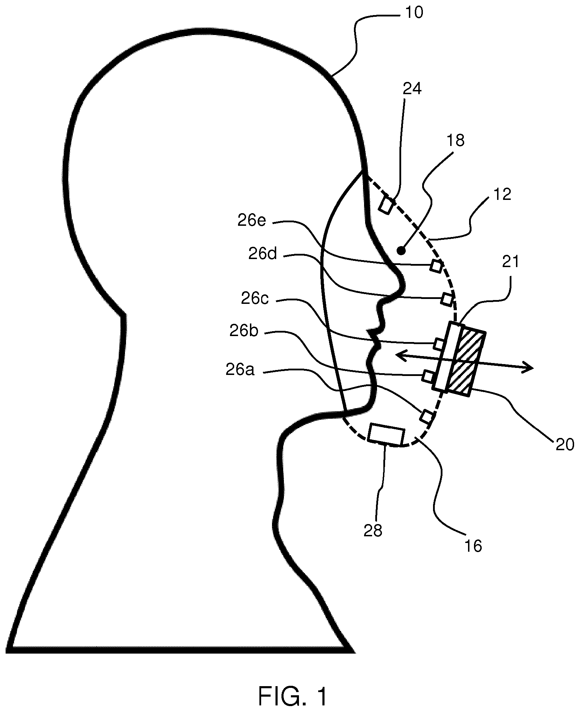

Fig. 1 shows a subject 10 wearing a face mask 12 which covers the nose and mouth of the subject. The purpose of the mask is to filter air before it is breathed in by the subject and to provide active control of the flow of air into an air chamber 18 (i.e. the mask chamber or mask volume) and/or out of the air chamber. An air pump arrangement 20 provides active flow control. The air pump arrangement may be a fan arrangement (of one or multiple fans) or it may be an arrangement of pump channels, as explained below.

A filter 21 is provided in series with the air pump arrangement 20.

The example shown has bidirectional air pumping.

Air is drawn in to the air chamber 18 by inhalation, and with assistance provided by the air pump arrangement.

During exhalation, air is expelled from the air chamber 18. In this example, exhalation is through the same air pump arrangement and hence also through the filter 21 (even though the expelled air does not need to be filtered).

The operation of the fan arrangement is preferably also timed with the breathing cycle of the user. For this purpose, a pressure sensor 24 is provided either for measuring the pressure inside the air chamber 18 or for measuring a differential pressure between the inside and outside of the air chamber.

Various mask designs are possible, with assisted inhalation and/or assisted exhalation, and with different filter designs. When there is both assisted inhalation and exhalation, there may be a single air pump arrangement or separate inhale and exhale air pumps.

The mask further comprises a sensor arrangement for distinguishing between a nasal breathing type and an oral breathing type. In the example shown, the sensor arrangement comprises a set of sensor modules 26a to 26e at different locations in the mask chamber 18, for example at different positions on the inside of the face mask 12. Each sensor module 26a to 26e comprises a temperature sensor and a relative humidity sensor.

When the user breathes out, the direction and spread of the exhaled air flow will depend on whether nasal breathing or oral breathing is taking place. The exhaled air flow has different temperature and relative humidity characteristics (typically body temperature of 37 degrees Celsius and 100% relative humidity) to the other air in the mask chamber. The set of sensor signals thus enables distinguishing between nasal and oral breathing. There may be only two sensors, one for detecting nasal breathing and one for detecting oral breathing. At the limit, there may be a single sensor module. It may for example be sufficient to sense only in one location to determine between nasal and oral breathing types (since there are only two possibilities).

The air pump arrangement 20 comprises at least a first flow area and a second flow area, in particular one area directed to the nose and one area directed to the mouth. The mask has a controller 28 for controlling the air pump arrangement to provide a flow though the first flow area and/or the second flow area in dependence on the detected breathing type.

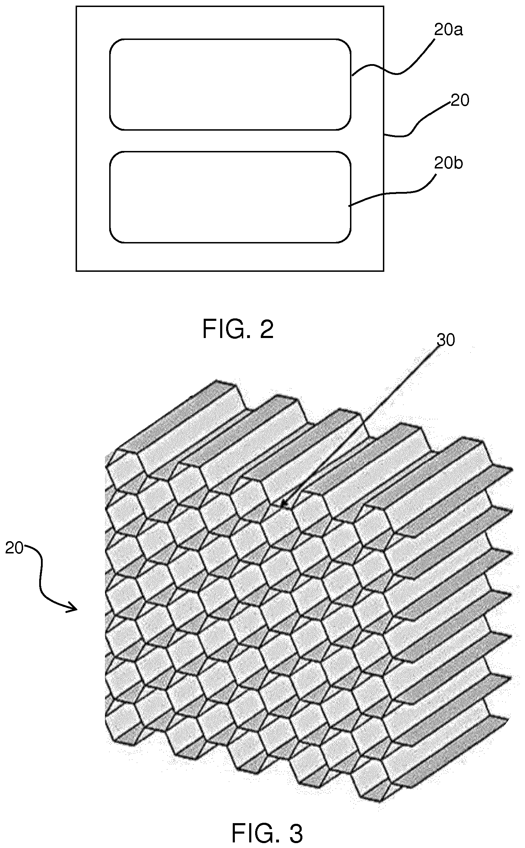

Fig. 2 shows the most basic arrangement for the air pump arrangement, in which there is a first flow area 20a and a second flow area 20b. The flow through the first flow area and the second flow area are independently controllable.

In this way, the first flow area 20a may be selected at one time to implement the air flow, and this area may be associated with nasal breathing. The first flow area 20a has a position selected so that it faces the nostrils. It may also have a flow direction towards the nostrils.

At another time ,the second flow area 20b may be selected to implement the air flow, and this area may be associated with oral breathing. The second flow area 20b has a position selected so that it faces the mouth. It may also have a flow direction towards the mouth.

The flow areas may generate flows which are parallel to each other, but they may have non-parallel flow directions.

For inhalation, the breathing assistance provided is most effective when the inward flow is directed to the mouth or nose according to the breathing type being used. Similarly, for exhalation, the ventilation is most effective in removing exhaled air (rather than other air in the mask chamber) when the exhaled flow is aligned with the outward flow stream provided by the air pump arrangement. Thus, the control of the flow area is of benefit for a mask with an inhalation pump (or fan) and/or for a mask with an exhalation pump (or fan).

In all cases, the mask design adapts the air pump arrangement to the type of breathing of the wearer of the mask.

In the example shown, the air pump arrangement is for controlling the passage of air into the mask chamber and out of the mask chamber. In this way, the flow is controlled during breathing in and breathing out, and different flow areas may be used for different parts of the breathing cycle, or else the same flow area may be used for both breathing in and out. Thus, the air pump arrangement may be configurable differently as between breathing in and breathing out, if desired.

Fig. 3 shows an air pump arrangement 20 the form of an array of pump channels 30. This provides a compact micropump arrangement. The pump channels may be grouped so that one group forms the area 20a and another group forms the area 20b. These groups may then be controlled as single units. However, the pump channels 30 may instead be independently controllable so that the flow areas may be controlled in a more dynamic way. For example, the flow rate may be controlled by selecting the number of flow channels which are in use.

The channels 30 may be grouped into rows and columns, with each area 20a, 20b comprising a set of rows or columns.

Each pump channel 30 may have a controllable pump flow direction. Thus, the single pump channel arrangement functions both as an inhale (inlet) and exhale (exhaust) pump. There may be only pump channels providing outward flow during exhalation (as determined by the controller) and only pump channels providing inward flow during inhalation, but there could also be inward and outward flow at the same time at different flow channels.

As shown in Fig. 3, every cell of the honeycomb comprises an individual pump cell and they may be controlled separately or connected for a simple implementation. One simple example is that the control terminals for the cells are connected together in each row, and different rows are separately controlled. For example, rows 1,2,3 and 4 could be controlled at the same time to make these rows act as intake units, whereas rows 5, 6, 7, and 8 could be controlled at the same time as discharge units. Each row will have several lines connected (for example 3) to a controller.

Typically, one terminal is connected to a positive voltage, another terminal is connected to a negative voltage, and a third terminal is connected to a control voltage that could be positive or negative.

By switching the third terminal, a diaphragm film of each single cell moves towards the first or second terminal by electrostatic attraction and repulsion. This generates the desired micro flow. By controlling the sequence of signals applied to the third terminal, the flow rate is controlled, and by switching the first and second terminals the direction of flow also can be controlled.

For a full implementation there could be more than three terminals for each cell for better performance. There are insulation layers between the rows.

By way of example, each cell is hexagonal with the first and second terminals on opposite faces, and the third terminal arranged to move between them.

The pump channels are all parallel in this example, but there could instead be pump channels with different directions in the different groups.

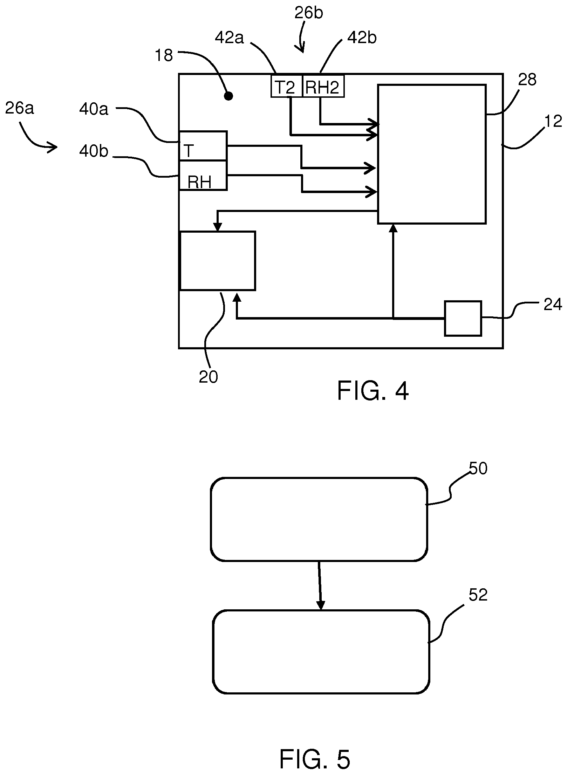

Fig. 4 shows one example of the components of the mask. The same components as in Fig. 1 are given the same reference numbers.

The example shows two sensor modules 26a, 26b. The first has a temperature sensor 40a and a relative humidity sensor 40b and the second has a temperature sensor 42a and a relative humidity sensor 42b.

There are of course many options for the shapes and locations of the areas 20a, 20b and the way they are directed.

The sensing modules may be separate units or they may be combined into the design of the air pump arrangement. The controller may determine the breathing type based on identification of the sensor module(s) which report the highest temperature and humidity and the sensor module(s) which report the lowest temperature and humidity.

The channels (or groups of channels) may then be controlled to be on with inward flow, on with outward flow or off.

Fig. 5 shows a method of controlling the mask.

In step 50, a nasal breathing type or an oral breathing type is identified.

In step 52, the air pump arrangement is controlled to provide a flow though a first flow area and/or a second flow area in dependence on the detected breathing type.

The micropump array comprises an array of channels. There may for example be tens or hundreds of pump channels. Each micropump for example comprises a piezoelectric actuator which is fixed to a diaphragm which forms a pump chamber. Multiple pump chambers may be connected in rows or columns.

This type and other types of micropump structure are known. For example, US 2009/0232683 discloses a micropump arrangement using piezoelectric actuators. US 2012/0304993 discloses the use of such a micropump within a breathing assistance device.

In general, micropumps are devices that can control and manipulate small fluid volumes, with functional dimensions (e.g. channel widths) in the micrometer or millimeter range.

The volumetric forces, such as weight or inertia, often become negligible within a micropump, whereas surface forces can dominate the pump behavior. There are mechanical and non-mechanical micropump designs. Mechanical systems contain moving parts, which are usually actuation and valve membranes or flaps. The driving force can be generated by utilizing piezoelectric, electrostatic, thermo-pneumatic, pneumatic or magnetic effects. Non-mechanical pumps function with electro-hydrodynamic, electro-osmotic, electrochemical or ultrasonic flow generation.

Any suitable micropump design may be used.

In the example above, the inlet (for inhalation assistance) and exhaust (for ventilation) is controlled by the air pump arrangement. Instead, exhalation may be through an outlet valve such as a check valve. This value may be closed during inhalation due to the low pressure in the air chamber. When the subject breathes out, air is exhausted through the outlet valve. This valve is then opened to enable easy exhalation, but is closed during inhalation. In this way, it is prevented that unfiltered air is drawn in. The timing of the outlet valve is then dependent on the breathing cycle of the subject. The outlet valve may be a simple passive check valve operated by the pressure difference across the mask. However, it may instead be an electronically controlled valve.

Thus, a design may have only an inhale air pump arrangement for assisted breathing, and for example for maintaining a positive pressure.

Similarly, an arrangement may have only an exhale air pump arrangement and the user may inhale through the filter manually. During exhalation, more air may be removed from the air chamber 18 than is exhaled so that additional air is supplied to the face through the inlet. This increases comfort due to lowering relative humidity and cooling. For example, a minimum exhaust flow rate may be provided for reducing the relative humidity inside the air chamber

The example above makes use of temperature and relative humidity measurement. Only one of these measurements may instead suffice. Also, other measurements may instead be made, such as force measurements for detecting the force of a flow of air breathed out towards a sensor.

The air pump arrangement may also be provided with the sensor arrangement. For example, a drive signal such as a current may depend on the external load on the pump channel (e.g. an air flow against the channel). Thus, the drive electronics of the air pump arrangement may be able to implement the sensing.

The invention may implemented with fans instead of a micropump, for example by having two inhale fans and/or two exhale fans.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A breathing mask comprising:
a mask body (12) for covering the nose and mouth of a user thereby defining a mask chamber (18);
a filter (21);
an air pump arrangement (20) for ventilating the mask chamber (18), wherein the air pump arrangement comprises a first flow area and a second flow area, wherein the flow through the first flow area and the second flow area are independently controllable; **characterized in that** the breathing mask further comprises:
a sensor arrangement (26a - 26e) for distinguishing between a nasal breathing type and an oral breathing type; and
a controller (28) for controlling the air pump arrangement to provide a flow though the first flow area and/or the second flow area for configuring a location and/or direction of air flow into and/or out of the mask chamber in dependence on the detected breathing type.

2. A mask as claimed in claim 1, wherein the air pump arrangement (20) is for controlling the passage of air into the mask chamber (18) and out of the mask chamber (18).

3. A mask as claimed in any preceding claim, wherein the sensor arrangement comprises a plurality of sensor modules (26a - 26e), at different locations within the mask chamber (18).

4. A mask as claimed in claim 3, wherein one sensor module is located at a position which, in use of the mask, is for detecting a nasal breath flow and one sensor module is located at a position which, in use of the mask, is for detecting an oral breath flow.

5. A mask as claimed in claim 3 or 4, wherein each sensor module comprises a temperature sensor (40a, 42a) and a relative humidity sensor (40b, 42b).

6. A mask as claimed in any preceding claim, wherein the air pump arrangement comprises an array of pump channels (30).

7. A mask as claimed in claim 6, wherein the pump channels (30) are controllable either individually or in groups.

8. A mask as claimed in claim 7, wherein each pump channel (30) has controllable pump flow direction.

9. A mask as claimed in claim 6, 7 or 8, wherein the pump channels comprise a first sub-array (20a) forming the first flow area and a second sub-array (20b) forming the second flow area.

10. A mask as claimed in any preceding claim, wherein the controller (28) is further adapted to control the air pump arrangement in synchronism with the breathing cycle of the user.

## Patentansprüche

1. Eine Atemmaske, die Folgendes umfasst:
ein Maskengehäuse (12) zum Abdecken von Nase und Mund des Benutzers, um eine Maskenkammer (18) zu bilden;
einen Filter (21);
eine Luftpumpenanordnung (20) zum Belüften der Maskenkammer (18), wobei die Luftpumpenanordnung einen ersten Strömungsbereich und einen zweiten Strömungsbereich umfasst, wobei die Strömung durch den ersten Strömungsbereich und den zweiten Strömungsbereich unabhängig voneinander gesteuert werden kann; die sich dadurch auszeichnet, dass die Atemmaske zudem Folgendes umfasst:
eine Sensoranordnung (26a - 26e) zum Unterscheiden zwischen einem nasalen Atemtyp und einem oralen Atemtyp; und
eine Steuerung (28) zum Steuern der Luftpumpenanordnung, um eine Strömung im ersten Strömungsbereich und/oder im zweiten Strömungsbereich bereitzustellen und abhängig von der erkannten Atmungsart eine Position und/oder eine Richtung für die Luftströmung in die und/oder aus der Maskenkammer zu konfigurieren.

2. Eine Maske gemäß Anspruch 1, wobei die Luftpumpenanordnung (20) zum Steuern der Luftströmung in die Maskenkammer (18) und aus der Maskenkammer (18) dient.

3. Eine Maske gemäß einem der vorhergehenden Ansprüche, wobei die Sensoranordnung an verschiedenen Positionen in der Maskenkammer (18) mehrere Sensormodule (26a - 26e) umfasst.

4. Eine Maske gemäß Anspruch 3, wobei sich ein Sensormodul an einer Position befindet, die beim Verwenden der Maske zum Erkennen einer nasalen Atmungsströmung dient, während sich ein anderes Sensormodul an einer Position befindet, die beim Verwenden der Maske zum Erkennen einer oralen Atmungsströmung dient.

5. Eine Maske gemäß Anspruch 3 oder 4, wobei die einzelnen Sensormodule einen Temperatursensor (40a, 42a) und einen Sensor für die relative Feuchtigkeit (40b, 42b) umfassen.

6. Eine Maske gemäß einem der vorhergehenden Ansprüche, wobei die Luftpumpenanordnung eine Anordnung von Pumpenkanälen (30) umfasst.

7. Eine Maske gemäß Anspruch 6, wobei die Pumpenkanäle (30) entweder einzeln oder in Gruppen gesteuert werden können.

8. Eine Maske gemäß Anspruch 7, wobei die einzelnen Pumpenkanäle (30) eine steuerbare Pumpenströmungsrichtung aufweisen.

9. Eine Maske gemäß Anspruch 6, 7 oder 8, wobei die Pumpenkanäle eine erste Teilanordnung (20a), die den ersten Strömungsbereich bildet, sowie eine zweite Teilanordnung (20b) umfassen, die den zweiten Strömungsbereich bildet.

10. Eine Maske gemäß einem der vorhergehenden Ansprüche, wobei die Steuerung (28) zudem die Luftpumpenanordnung synchron mit dem Atemzyklus des Benutzers steuert.

## Revendications

1. Masque respiratoire comprenant :
un corps de masque (12) destiné à la couverture du nez et de la bouche d'un utilisateur définissant ainsi une chambre de masque (18) ;
un filtre (21) ;
un agencement de pompe à air (20) destiné à la ventilation de la chambre de masque (18), dans lequel l'agencement de pompe à air comprend une première zone d'écoulement et une seconde zone d'écoulement, dans lequel les écoulements à travers la première zone d'écoulement et la seconde zone d'écoulement peuvent être commandés indépendamment ;
**caractérisé en ce que** le masque respiratoire comprend en outre :
un agencement de capteur (26a-26e) destiné à la distinction entre un type de respiration nasale et un type de respiration buccal ; et
un dispositif de commande (28) pour commander l'agencement de pompe à air pour fournir un écoulement à travers la première zone d'écoulement et/ou la seconde zone d'écoulement pour configurer un emplacement et/ou une direction d'écoulement d'air dans et/ou hors de la chambre de masque en fonction du type de respiration détecté.

2. Masque selon la revendication 1, dans lequel l'agencement de pompe à air (20) est destiné à la commande du passage de l'air dans la chambre de masque (18) et hors de la chambre de masque (18).

3. Masque selon l'une quelconque des revendications précédentes, dans lequel l'agencement de capteur comprend une pluralité de modules de capteur (26a-26e), à différents emplacements à l'intérieur de la chambre de masque (18).

4. Masque selon la revendication 3, dans lequel un module capteur est situé à une position, laquelle, lors de l'utilisation du masque, sert à détecter un écoulement respiratoire nasal et un module capteur est situé à une position, laquelle, lors de l'utilisation du masque, sert à détecter un écoulement respiratoire buccal.

5. Masque selon la revendication 3 ou 4, dans lequel chaque module de capteur comprend un capteur de température (40a, 42a) et un capteur d'humidité relative (40b, 42b).

6. Masque selon l'une quelconque des revendications précédentes, dans lequel l'agencement de pompe à air comprend un réseau de canaux de pompe (30).

7. Masque selon la revendication 6, dans lequel les canaux de pompe (30) peuvent être commandés individuellement ou en groupes.

8. Masque selon la revendication 7, dans lequel chaque canal de pompe (30) comporte une direction d'écoulement de pompe pouvant être commandée.

9. Masque selon la revendication 6, 7 ou 8, dans lequel les canaux de pompe comprennent un premier sous-réseau (20a) formant la première zone d'écoulement et un second sous-réseau (20b) formant la seconde zone d'écoulement.

10. Masque selon l'une quelconque des revendications précédentes, dans lequel le dispositif de commande (28) est en outre conçu pour commander l'agencement de pompe à air par synchronisation avec le cycle respiratoire de l'utilisateur.
